# FASCICULE DE BREVET EUROPEEN

(11) **EP 3 579 823 B1**
(45) Date de publication et mention de la délivrance du brevet: **04.08.2021**
(21) Numéro de dépôt: 18712506.7
(22) Date de dépôt: 09.02.2018
(51) Int. Cl.: A61K 9/48, A61K 39/35, A61J 3/07, A61K 39/00, A61K 35/35, A61K 31/7016, A61K 36/48, A61K 45/06, A61P 37/08

(54) **GÉLULE À LIBÉRATION GASTRO-INTESTINALE DESTINÉE À ÊTRE UTILISÉE DANS UNE MÉTHODE PERMETTANT DE DÉSENSIBILISER ET/OU D'INDUIRE UNE TOLÉRANCE CHEZ UN SUJET ALLERGIQUE À L'ARACHIDE**
KAPSEL ZUR GASTROINTESTINALEN FREISETZUNG FÜR DIE VERWENDUNG IN EINEM VERFAHREN ZUR DESENSIBILISIERUNG UND VERTRÄGLICHKEITSINDUZIERUNG BEI EINEM PATIENTEN MIT ERDNUSSALLERGIE
GASTROINTESTINAL RELEASE CAPSULE FOR USE IN A METHOD FOR DESENSITISING AND/OR INDUCING TOLERANCE IN A PATIENT WITH A PEANUT ALLERGY

(30) Priorité: 10.02.2017 FR 1751101
(43) Date de publication de la demande: 18.12.2019
(73) Titulaire: Centre Hospitalier et Universitaire de Clermont-Ferrand, 63000 Clermont-Ferrand (FR); Université Clermont Auvergne, 63000 Clermont-Ferrand (FR)
(72) Inventeur: FAUQUERT, Jean-Luc, 63110 Beaumont (FR); EVRARD, Bertrand, 63122 Ceyrat (FR); MICHAUD, Elodie, 63410 Charbonnières les Varennes (FR); MERLIN, Etienne, 63400 Chamalières (FR)
(74) Mandataire: Vidon Brevets & Stratégie
(86) Numéro de dépôt international: PCT/EP2018/053344
(87) Numéro de publication internationale: WO 2018/146274

(56) Documents cités:
- WO-A2-2016/134291
- US-A1- 2014 271 721
- Anonymous: "Oral Desensitization to Peanut in Peanut-Allergic Children and Adults Using Characterized Peanut Allergen OIT", , 23 juin 2016 (2016-06-23), XP055411613, Extrait de l'Internet: URL:https://clinicaltrials.gov/ct2/show/NC T01987817 [extrait le 2017-09-29]
- Anonymous: "OIT Gets Ready for Prime Time - Allergic Living", , 18 juillet 2016 (2016-07-18), XP055411614, Extrait de l'Internet: URL:https://allergicliving.com/2016/07/18/ oit-gets-ready-prime-time/ [extrait le 2017-09-29]

## Description

### 1. Domaine de l'invention

Le domaine de l'invention est celui de la composition pharmaceutique et des indications médicales. Plus précisément, l'invention concerne une gélule à libération gastro-intestinale comprenant un cœur et une enveloppe, ledit cœur comprenant une composition d'arachides. Ladite gélule est destinée à être utilisée par ingestion pour une libération gastro-intestinale permettant une méthode d'immunothérapie orale par libération gastro-intestinale pour désensibiliser, et/ou d'induire une tolérance à l'arachide chez un sujet allergique à l'arachide. Ladite gélule est également destinée à diagnostiquer une allergie à l'arachide chez un sujet suspect d'allergie à l'arachide.

### 2. Art antérieur

L'allergie à l'arachide touche 1% à 2 % de la population pédiatrique. Elle occupe la première place des allergies alimentaires chez l'enfant de plus de 3 ans. Contrairement à l'allergie à l'œuf de poule ou au lait de vache, l'allergie à l'arachide ne disparaît que dans 20 % des cas (Rancé et al, Clin. Exp. Allergy, 2005, 35 :167-172). Elle est potentiellement grave : l'ingestion de petites quantités d'arachide peut en effet conduire à des réactions graves voire létales. Ces réactions sont consécutives d'un mécanisme médié par les Immunoglobulines de type E (IgE), tant pour la réaction princeps - qui conduit au diagnostic d'allergie à l'arachide - que pour les réactions ultérieures et imprévisibles lors d'ingestions accidentelles ou masquées.

Ces réactions allergiques survenant après l'ingestion de l'allergène comprennent des manifestations respiratoires (toux spasmodique, dyspnée, crise d'asthme), cutanées (urticaire, chémosis, angio-oedème atteignant la peau ou les muqueuses des voies aériennes supérieures responsable de dyspnée laryngée), digestives (douleurs abdominales, nausées, vomissements, diarrhées) ou générales, allant du simple malaise à l'anaphylaxie aiguë avec ou sans collapsus cardio-vasculaire générateur de choc. Ces symptômes sévères sont en particulier rapportés à la fraction allergénique ara h2 de l'arachide, mais aussi aux autres allergènes de stockage de l'arachide tels que ara h1, ara h3 et ara h6. Il peut aussi s'agir d'atteinte des voies aériennes supérieures (rhinite, conjonctivite, ou syndrome oral avec prurit, érythème ou œdème atteignant la cavité buccale, voire le pharynx) ou digestives supérieures dont l'œsophagite à éosinophiles. Le dégoût vis-à-vis de l'arachide fait aussi partie des signes classiques de la maladie. Ces signes sont souvent associés et différents stades de gravité ont été décrits dans la littérature (Lieberman P, Nicklas RA, Oppenheimer J et al, The diagnosis and management of anaphylaxis:an updated practice parameter. J. Allergy Clin. Immunol. 2010;126:477-80). Les enfants allergiques à l'arachide sont donc exposés en permanence au risque anaphylactique qui fait régulièrement l'objet de mises au point dans la littérature médicale (EAACI Guidelines on allergen immunotherapy: IgE-mediated food allergy. Allergy. 2017 Sep 27. doi: 10.1111/all.13319).

Le diagnostic d'allergie à l'arachide est basé sur la présence d'une histoire clinique évocatrice de réaction après ingestion, chez un patient sensibilisé par un contact préalable avec l'arachide. La positivité d'un test cutané et d'un dosage d'IgE spécifiques sériques à l'arachide témoigne de la sensibilité du patient à l'arachide. En cas de doute sur la responsabilité de l'arachide, la pratique d'un test de provocation par voie orale (TPO) est nécessaire pour étayer la relation entre l'allergène et la réaction qu'il provoque. Ainsi le TPO est considéré comme « l'étalon-or » pour poser le diagnostic de certitude de l'allergie à l'arachide, surtout s'il est pratiqué en double aveugle et contre placebo (DACP). Il est aussi utilisé pour déterminer le seuil réactogène à l'arachide et permettre ainsi d'adapter les mesures d'éviction. Sa pratique implique des mesures particulières de prévention. Il doit être réalisé dans un service habitué à sa réalisation (protocole précis, mesures thérapeutiques anticipées) et apte à faire face à une réaction suraiguë (service de réanimation à proximité).

Pour pallier l'effet nocebo, le recours à la technique du double aveugle (patient et médecin aveugles) est recommandé. Le goût et l'odeur spécifiques de l'arachide sous forme native rendent cependant cette pratique difficile, considérant notamment la pratique consistant à mélanger la composition d'arachide au bol alimentaire. A titre de précision, l'effet nocebo est le pendant négatif de l'effet placébo. Il est de nature psychologique. Plus précisément, il apparaît lorsque le patient développe des effets secondaires nuisibles même en consommant une substance inerte. Le développement de ces effets secondaires néfastes vient du fait que le patient est convaincu que la substance qu'on lui présente est nuisible ou qu'elle va avoir des effets nuisibles sur sa personne. Dans le cas de l'arachide, les patients seraient prompts à développer des allergies s'ils pensent être allergiques à l'arachide, ou à tout le moins à déclencher une réaction allergique à des seuils bien moindres.

La prise en charge thérapeutique de l'allergie à l'arachide était jusqu'à ces dernières années limitée à l'éviction alimentaire stricte de l'arachide. Le constat de la fréquence plus élevée des réactions graves après ingestion accidentelle d'arachide, chez les patients suivant une éviction stricte comparée à celle des enfants qui suivent une éviction relative, a conduit à pratiquer ces dernières années des protocoles d'induction de tolérance à l'arachide, sans résultat probant jusqu'à présent. Des études ont été mises en place pour tester les concepts de désensibilisation, voire de tolérance à l'arachide, par différentes techniques, sans consensus jusqu'à présent au sein de la communauté médicale.

Les méthodes d'immunothérapie ont pour but de réduire le risque de réaction sévère voire mortelle après ingestion accidentelle d'arachide et d'améliorer la qualité de vie des patients atteints de cette maladie. Les méthodes les plus répandues consistent en l'administration d'arachide par voie orale. Elles comportent une phase d'incrémentation des ingesta qui a pour but une désensibilisation à l'allergène (qui impose au patient de continuer à ingérer des petites d'allergène pour être protégé), puis une phase de tolérance qui protège le patient alors qu'il n'ingère plus l'allergène au quotidien. Tel que rapporté dans de nombreuses publications scientifiques, ces inductions de tolérance par voie orale sont conventionnellement réalisées par mélange de la composition d'arachide ou équivalent au bol alimentaire. Si la composition d'arachide est formulée sous la forme d'une gélule ou d'une capsule, cette gélule ou capsule est alors ingérée sous forme ouverte, pour permettre le mélange de la composition d'arachide au bol alimentaire. Par cela, la communauté médicale préconisait une approche basée sur des éléments physiopathologiques. En effet, il est communément reconnu qu'il est préféré, voire indispensable, d'exposer les cellules immunitaires de la cavité buccale et de l'œsophage à la composition d'arachide.

En l'état actuel de la littérature, l'immunothérapie n'est pas complètement validée comme une technique de traitement de l'allergie à l'arachide, pour des raisons tenant à l'acceptabilité du traitement et la compliance des patients, et surtout aux effets secondaires de ces techniques d'immunothérapie orale, notamment aux effets secondaires et/ou indésirables, y inclus les manifestations oropharyngées et digestives, dont l'œsophagite à éosinophiles, et aux réactions anaphylactiques.

Des méthodes alternatives basées sur d'autres voies de pénétration de l'allergène, notamment par voie sublinguale ou épicutanée, ont été récemment développées. Pour ce qui concerne les méthodes basées sur l'application épicutanée sous forme de patchs, la pénétration transdermique de l'allergène arachide est moindre que celle de l'immunothérapie par voie orale et peut varier en fonction de la qualité fonctionnelle de la peau (déficit en filaggrine mis en évidence chez les sujets atopiques, âge, sexe, race, etc.). Après ingestion accidentelle, les doses qui pénètrent dans l'organisme sont souvent élevées. A noter cependant qu'aucun résultat probant n'a été obtenu à présent.

Il existe donc un besoin de proposer un moyen simple, efficace et sécurisé de désensibiliser les patients à l'arachide.

### 3. Objectifs de l'invention

L'invention a notamment pour objectif de pallier ces inconvénients de l'art antérieur, notamment en matière d'acceptabilité, d'efficacité et de sécurité par la réduction des effets secondaires et/ou indésirables.

Plus précisément, un objectif de l'invention est de fournir, dans au moins un mode de réalisation, une composition permettant de désensibiliser et/ou d'induire une tolérance chez les sujets allergiques à l'arachide.

Un autre objectif de l'invention est de proposer, dans au moins un mode de réalisation, une composition permettant de diagnostiquer une allergie chez un sujet.

Un autre objectif de l'invention est de proposer, dans au moins un mode de réalisation, une composition permettant de désensibiliser un sujet allergique à l'arachide de manière exhaustive et définitive.

Un autre objectif de l'invention est de proposer, dans au moins un mode de réalisation, une composition destinée à être utilisée dans une méthode permettant de désensibiliser et/ou d'induire une tolérance chez un sujet allergique à l'arachide, tout en masquant efficacement le goût, la couleur et l'odeur, en éliminant ainsi le dégoût alimentaire vis à vis de cet allergène.

Un autre objectif de l'invention est de proposer, dans au moins un mode de réalisation, une composition destinée à être utilisée dans une méthode permettant de désensibiliser et/ou d'induire une tolérance chez un sujet allergique à l'arachide, en réduisant les effets secondaires, notamment le risque de réactions oro-pharyngées (syndromes oraux), d'œsophagite à éosinophiles, de réaction anaphylactique et autres effets indésirables.

Un autre objectif de l'invention est de proposer, dans au moins un mode de réalisation, une composition destinée à être utilisée dans une méthode permettant de désensibiliser et/ou d'induire une tolérance chez un sujet allergique à l'arachide, tout en minimisant la survenue de l'effet nocebo.

Un autre objectif de l'invention est de proposer, dans au moins un mode de réalisation, une composition destinée à être utilisée dans une méthode permettant de désensibiliser et/ou d'induire une tolérance chez un sujet allergique à l'arachide âgé de 1 à 18 ans.

### 4. Exposé de l'invention

Ces objectifs, ainsi que d'autres qui apparaîtront par la suite, sont atteints à l'aide d'une gélule ingérée par voie orale à libération gastro-intestinale, destinée à être utilisée pour désensibiliser un sujet allergique à l'arachide et/ou pour induire une tolérance à l'arachide chez un sujet allergique à cet allergène, ladite gélule comprenant une enveloppe et un cœur, ledit cœur comprenant une composition comprenant de l'arachide, au moins une huile, au moins un premier excipient pulvérulent, et optionnellement au moins un second excipient prébiotique. Pour ce faire, ladite gélule est fournie et ingérée sous une forme non-ouverte. En d'autres termes, la présente invention concerne une gélule sous forme non-ouverte à libération gastro-intestinale (gélule non-ouverte à libération gastro-intestinale) ingérée par voie orale, destinée à être utilisée pour désensibiliser un sujet allergique à l'arachide et/ou pour induire une tolérance à l'arachide chez un sujet allergique à cet allergène, ladite gélule comprenant une enveloppe et un cœur, ledit cœur comprenant une composition comprenant de l'arachide, au moins une huile, au moins un premier excipient pulvérulent, et optionnellement au moins un second excipient prébiotique.

Par « gélule », on entend une forme galénique telle qu'une gélule ou une capsule, à enveloppe dure ou molle, contenant en son cœur une quantité fixe de composition d'arachide ainsi qu'une quantité fixe d'excipients. La composition d'arachide est donc isolée au sein de la gélule, et n'est rendue disponible qu'après ouverture de la gélule et/ou rupture de l'enveloppe. Les gélules selon l'invention se distinguent donc des formes ingérées isolement ou au sein du bol alimentaire sous forme de comprimés non-enveloppés, des poudres et suspensions liquides, en ce que le principe actif (à savoir l'arachide présentement) n'est pas immédiatement disponible. Les gélules selon l'invention se distinguent également des gélules ingérées sous forme ouverte, en ce que le principe actif (à savoir l'arachide présentement) n'est pas immédiatement disponible, c'est-à-dire en ce que le principe actif n'entre pas en contact avec la muqueuse buccale.

Par « à libération gastro-intestinale », on entend une gélule dont l'ouverture de la gélule et/ou la rupture de l'enveloppe se produit au niveau de l'estomac et/ou de l'intestin grêle. En d'autres termes, l'intégrité de la gélule est préservée lors de l'ingestion par la bouche et son passage via l'œsophage. L'ouverture de la gélule et/ou la rupture de l'enveloppe se produit sous l'action mécanique et physiologique de l'estomac et/ou de l'intestin grêle. En cela, la gélule selon la présente invention est ingérée sous forme non-ouverte.

On calcule le pourcentage massique par la formule suivante : Pourcentage massique d'un composé A (%) = (masse de A/ masse totale de la composition) × 100.

Ainsi, l'invention repose sur une approche tout à fait nouvelle et originale d'administrer par voie orale de l'arachide tout en masquant l'odeur, la couleur et la saveur caractéristiques de l'arachide et en minimisant le risque de réaction allergique au niveau du tractus digestif haut ou de choc anaphylactique.

En particulier, la gélule selon l'invention est destinée à être utilisée dans une méthode permettant de désensibiliser et/ou de rendre tolérant (induire une tolérance chez) un sujet allergique, tout en évitant le contact avec les muqueuses buccale, pharyngée et de l'œsophage, lesquelles sont riches en cellules immunitaires et en cellules présentatrices de l'antigène notamment.

Les cellules présentatrices de l'antigène sont des cellules du système immunitaire qui présentent à leur surface un fragment antigénique de la molécule ou de l'organisme étranger, ici l'allergène. Ces cellules peuvent être des monocytes, des macrophages, des lymphocytes B ou des cellules dendritiques. Schématiquement, cette présentation de l'antigène par des cellules immunitaires permet une activation spécifique des lymphocytes T qui vont reconnaître l'épitope de la molécule ou de l'organisme étranger(e) pour pouvoir ensuite l'éliminer.

Les inventeurs proposent donc de shunter la cavité buccale, le pharynx et l'œsophage, et leur système immunitaire réactif pour éviter la survenue de réaction allergique chez le patient à chaque tentative de désensibilisation, ce qui mettrait de fait un terme à la démarche thérapeutique. En effet, les patients allergiques à l'arachide déclenchent des réactions plus ou moins violentes en cas de mise en contact. L'imprévisibilité de ces réactions et de leur intensité implique de s'entourer de précautions particulières lors d'un protocole d'immunothérapie à l'arachide. Les doses ingérées sont très faibles en début de protocole et augmentent lentement pendant une période longue pour éviter le risque d'effets secondaires. De plus, la survenue d'une réaction allergique répétée ou sévère implique l'arrêt du protocole de désensibilisation. L'évitement d'une réaction allergique trop violente est donc crucial pour la poursuite et le succès d'un procédé de diagnostic d'une allergie et/ou de désensibilisation aux arachides. En plus de ces réactions aigues à l'ingestion, le shunt décrit ci-dessus permettra de réduire le risque de survenue de manifestations chroniques de l'allergie, en particulier au niveau local de l'œsophagite à éosinophiles. A noter que cette approche va à l'encontre de nombreuses approches actuellement pratiquées, qui consiste à mélanger la composition d'arachide avec le bol alimentaire.

Les risques de non-observance du protocole par le sujet, et son interruption prématurée sont plus d'autant importants que cette phase est prolongée. Or, l'ingestion de la gélule sous forme fermée diminue les risques de non-observance et d'interruption du protocole. La réduction des effets secondaires et/ou indésirables et le masquage du goût contribuent ainsi à une bonne compliance du sujet vis-à-vis du protocole.

En outre, l'ingestion de la gélule sous forme fermée limite l'exposition des allergènes à l'acide gastrique, ce qui contribue à en préserver l'intégrité de leur structure tridimensionnelle.

Par ailleurs, la muqueuse de l'intestin grêle est dotée de nombreuses cellules présentatrices d'antigène spécialisées, notamment de phagocytes mononucléés à type de macrophages ou de cellules dendritiques. Les cellules dendritiques intestinales constituent ainsi un ensemble de sous-populations hétérogènes exprimant de façon différentielle un certain nombre de marqueurs phénotypiques, dont CD103, CD11b, CD172a/SIRPα et CX3CR1. Ces sous-populations ont des caractéristiques fonctionnelles différentes, en particulier certaines seraient fondamentales dans la mise en place de la tolérance orale aux antigènes alimentaires, notamment les cellules dendritiques migratrices CD103⁺CD11b⁻ et dans une moindre mesure CD103⁺CD11b⁺. En effet, en condition homéostasique, après migration dans les ganglions lymphatiques mésentériques, ces cellules auraient la capacité de produire de l'acide rétinoïque grâce à une enzyme, la RALDH2 (retinaldehyde dehydrogenase 2), ainsi que du TGF-β et exprimeraient l'IDO (indoléamine 2,3-dioxygénase). L'ensemble de ces éléments permettraient la conversion des lymphocytes T naïfs en T régulateurs périphériques CD4⁺CD25⁺FoxP3 et leur homing dans la muqueuse intestinale. Ces lymphocytes seraient ensuite eux-mêmes impliqués dans l'inhibition des réponses Th2 et IgE spécifiques aux antigènes alimentaires, et dans la promotion des réponses IgG4. Les cellules dendritiques pourraient également favoriser le fonctionnement d'autres lymphocytes T régulateurs, de type Th3 par exemple, ainsi que celui des ILC (Innate Lymphoïd cells) ou des lymphocytes B régulateurs. Une perturbation du fonctionnement des cellules dendritiques pourra ainsi rompre l'équilibre délicat immunité vs tolérance, générant l'apparition d'une réponse de type Th2 et d'une hypersensibilité aux allergènes alimentaires. La gélule selon l'invention permet de libérer l'arachide au niveau de l'intestin grêle et de mettre en contact ces cellules dendritiques intestinales avec l'antigène contenu dans la composition d'arachide. Ainsi, en ciblant ces cellules dendritiques intestinales (sans l'intervention des cellules présentatrices de l'oropharynx), notre immunothérapie « à libération gastro-intestinale » joue directement sur les cellules supportant réellement *in vivo* l'induction de tolérance orale et permet d'optimiser notre protocole d'immunothérapie. A noter que le contact entre les cellules dendritiques intestinales et les allergènes pourra avoir lieu selon trois voies préférentielles, 1) par contact direct grâce à l'émission d'extensions cytoplasmiques par les cellules dendritiques CX3CR1+ et CD11b+ de la *Lamina propria* passant entre les cellules épithéliales et capturant les allergènes alimentaires directement dans la lumière intestinale ; 2) par contact indirect suite à la transcytose des allergènes au travers des cellules M situées au-dessus des plaques de Peyer; 3) par contact indirect via les cellules épithéliales intestinales capables d'internaliser les allergènes à leur pôle apical, de sécréter ensuite un environnement cytokinique tolérogène à leur pôle basal au contact des cellules dendritiques, ainsi que des exosomes portant des peptides allergéniques.

Par conséquent, la stimulation des cellules présentatrices de l'antigène au niveau de l'intestin grêle est une voie intéressante pour stimuler le système immunitaire du patient en limitant les risques de réaction allergique. En évitant le contact avec les muqueuses buccale, pharyngée et de l'œsophage, la composition selon l'invention permet d'une part de rendre possible la désensibilisation, et d'autre part d'alléger les précautions prises lors des tests de désensibilisation ou de l'ingestion du traitement.

Le masquage du goût, de la couleur et de l'odeur caractéristiques de l'arachide permet en outre de réduire considérablement, sinon de supprimer, l'effet nocébo. En effet, il est notable que les patients souffrant d'allergie à l'arachide présentent une aversion alimentaire qui constitue un symptôme caractéristique de la maladie. En ingérant de façon consciente et visible de l'arachide, dont ils connaissent l'effet délétère, ils sont susceptibles de développer des symptômes en tout point identiques à ceux d'une réaction allergique. Selon l'invention, la gélule permet aussi de respecter au mieux le principe du test en double aveugle.

Si nécessaire, tel qu'illustré présentement, l'arachide peut être conditionnée au préalable. Par exemple, l'arachide peut être conditionnée sous forme de pâte, par exemple par ajout d'une huile. La pâte d'arachide constituera alors la matière première de la composition d'arachide.

La présence d'un premier excipient pulvérulent est utile en ce qu'il permet d'obtenir une composition d'arachide sous forme pulvérulente (poudre), indépendamment de la matière première utilisée, telle qu'une pâte d'arachide. Cette forme facilite la préparation et le conditionnement de la composition d'arachide.

La présence d'un second excipient prébiotique permet de renforcer la flore commensale et de contribuer à renforcer l'immunité du sujet traité.

La formulation de la composition d'arachide avec ces premier et second excipients permet également de modifier l'absorption de l'arachide par la muqueuse intestinale.

La gélule ainsi optimisée permet de garder une présentation de l'allergène sous forme intacte et de répondre ainsi à l'objectif d'immunothérapie à l'arachide.

La gélule selon l'invention comprend préférentiellement une composition d'arachides grillées, très préférentiellement une composition d'arachides grillées et non-dégraissées.

La gélule selon l'invention comprend préférentiellement entre 10 mg et 1000 mg, très préférentiellement entre 10 mg et 750 mg, encore très préférentiellement entre 10 mg et 500 mg d'arachide.

Ladite composition selon l'invention peut comprendre entre 5% et 70% d'arachide, par rapport au poids total de la composition. En outre, ladite composition peut comprendre entre 15% et 55% du premier excipient pulvérulent, par rapport au poids total de la composition. De même, ladite composition peut comprend entre 10% et 75% du second excipient prébiotique, si présent, par rapport au poids total de la composition.

La gélule peut prendre différentes tailles. La taille de la gélule peut être adaptée à sa masse, tout en respectant la notion d'aveugle pharmaceutique. Ainsi, en ce qui concerne les principes actifs, excipients et autres composés, leurs proportions respectives pourront être ajustées de telle sorte à ce que la dose appropriée de principes actifs soit ingérée par le sujet à traiter, en fonction de l'étape en cours.

De manière avantageuse, la composition d'arachide comprend entre 10% et 40%, préférentiellement entre 10% et 30%, très préférentiellement entre 15% et 25%, de protéines allergéniques par rapport au poids total de ladite composition. En effet, les inventeurs ont mis en évidence qu'il était préférable, pour éviter les réactions allergiques au cours du protocole d'immunothérapie, et afin d'assurer une désensibilisation effective, d'utiliser des arachides ayant une proportion limitée en protéines allergéniques, c'est-à-dire inférieure à 40%, en lieu et place d'arachides ayant une proportion élevée en protéines allergéniques.

En corollaire, ladite composition en arachide comprend entre 10% et 15% des protéines totales pour la fraction allergénique Ara h1, entre 2% et 10% des protéines totales pour la fraction allergénique Ara h2 et entre 10% et 20% des protéines totales pour la fraction allergénique Ara h3 ; préférentiellement environ 12% des protéines totales pour la fraction allergénique Ara h1, environ 6% des protéines totales pour la fraction allergénique Ara h2 et environ 15% des protéines totales pour la fraction allergénique Ara h3, en poids de la fraction protéique de ladite composition. Cette composition particulière en allergènes, présents dans des proportions limitées permet de réunir les fractions allergéniques les plus fréquentes parmi la grande diversité des variétés d'arachide. Ainsi, la gélule selon l'invention présente un caractère exhaustif et universel pour la désensibilisation à l'allergie aux arachides. Il est en effet intéressant qu'un patient/sujet puisse être désensibilisé à différentes fractions allergéniques simultanément, de sorte qu'il puisse reprendre une vie normale sans se soucier de la variété d'arachide utilisée dans un plat ou ayant potentiellement contaminé le produit alimentaire.

Ladite gélule comprend préférentiellement une huile étant de l'huile de tournesol. L'allergie à l'huile de tournesol est exceptionnelle et n'expose pas au risque de réaction sévère. Il n'est en effet pas rare que les patients allergiques à l'arachide présentent d'autres sensibilisations ou allergies alimentaires, et en particulier à l'huile de noix, de noisette voire de colza. Ladite composition peut comprendre entre 0,5% et 5% de ladite huile, par rapport au poids totale de ladite composition.

Ladite gélule comprend un premier excipient pulvérulent pouvant être le phosphate tricalcique, très préférentiellement un premier excipient pulvérulent choisi parmi le phosphate tricalcique bêta, le phosphate tricalcique alpha, et leur mélange ; alternativement le phosphate tricalcique bêta ou le phosphate tricalcique alpha. La formulation de la composition d'arachide avec le phosphate tricalcique est avantageuse, en comparaison des autres excipients pulvérulents. En effet, le phosphate tricalcique est un adjuvant potentiel de l'immunité. En particulier, le phosphate tricalcique possède des propriétés immunomodulatrices intéressantes, contrairement aux autres formes de phosphate. Cette fonction contribue donc à la désensibilisation du sujet traité.

Ladite gélule peut comprendre en outre un second excipient prébiotique. Ledit second excipient prébiotique est préférentiellement le lactose, très préférentiellement le lactose monohydraté. La formulation de la composition d'arachide avec un second excipient prébiotique, notamment le lactose, est avantageuse en ce que ledit second excipient prébiotique contribue à améliorer la qualité de la flore commensale digestive, laquelle est essentielle en ce qu'elle contribue grandement à « l'éducation » du système immunitaire, et en ce qu'elle est un facteur essentielle dans la limitation de la survenue de phénomènes allergiques (à l'échelle de la population).

Ladite gélule selon l'invention comprend entre 20 mg et 315 mg du premier excipient pulvérulent et/ou entre 0 mg et 100 mg du second excipient prébiotique.

L'enveloppe de ladite gélule est préférentiellement opaque. Une enveloppe opaque permet d'éviter que le patient et/ou le praticien ne distingue la gélule contenant la composition d'arachides d'un placebo par exemple. L'enveloppe peut comprendre un composé choisi parmi le groupe consistant en la gélatine (d'origine animale ou marine), l'hydroxypropylmethyl de cellulose (HPMC), le pullulan ou leurs mélanges. A ce titre, la gélule selon l'invention peut également comprendre un opacifiant et/ou des colorants dans son enveloppe, dans la mesure de leur inertie au plan allergénique.

L'enveloppe de la gélule peut comprendre un corps et une tête, la tête étant fixée sur le corps pour refermer la gélule. En particulier, la gélule fermée peut avoir une longueur comprise entre 5 mm et 30 mm. La tête de l'enveloppe peut présenter une longueur comprise entre 5 mm et 15 mm et un diamètre compris entre 4,5 mm et 10 mm. Le corps de l'enveloppe peut présenter une longueur comprise entre 5 mm et 25 mm et un diamètre compris entre 4,5 mm et 10 mm.

La gélule telle que définie précédemment peut être utilisée pour la détermination du seuil de réaction d'une allergie à l'arachide. Ce seuil est déterminé in vivo par la pratique d'un test de provocation. Celui-ci justifie des mesures de prévention particulières et une équipe habituée ainsi que la présence d'un service de réanimation à proximité. La survenue d'une réaction clinique objective, même isolée, définit le seuil de reactivité (tableau 4), (Rancé F., Deschildre A, Villard-Truc F, Gomez SA, Paty E, Santos C, Couderc L, Fauquert JL, De Blic J, Bidat E, Dupont C, Eigenmann P, Lack G, Scheinmann P, SFAIC and SP2A workgroup on OFC in children. Oral food challenge in children : an expert review. Position paper of the Section of Pediatrics of the French Society of Allergology and Clinical Immunology (SFAIC) and of the Pediatric Society of Pulmunology and Allergology (SP2A). Eur Ann Allergy Clin Immunol 2009;41;2:35-49.).

Le diagnostic de l'allergie à l'arachide doit être préférentiellement basé sur la pratique d'un test de provocation orale (TPO) en double aveugle au cours duquel de l'arachide ou ses allergènes sont administrés au patient. L'odeur et la saveur caractéristique de l'arachide rendent le respect du double aveugle ou le diagnostic difficile. Par ailleurs, la possibilité qu'une réaction survienne ou soit intensifiée par la connaissance de la présence de l'allergène n'est jamais à exclure. La gélule et son ingestion sous forme fermée (non-ouverte) - en dissimulant l'aspect, l'odeur, la couleur et la saveur de son contenu - permet d'éviter tous ces inconvénients. De plus, la présence d'au moins un premier excipient pulvérulent et optionnellement d'au moins un second excipient permet d'améliorer son absorption intestinale.

L'invention concerne tout particulièrement la gélule telle que définie précédemment pour son utilisation dans une méthode permettant de désensibiliser et/ou de rendre tolérant (induire une tolérance chez) un sujet allergique à l'arachide. La démarche de désensibilisation peut également être désignée par les expressions « immunothérapie spécifique » ou « vaccinothérapie allergénique ». La désensibilisation consiste à réhabituer l'organisme à un allergène progressivement, par le biais d'une administration de doses croissantes d'une composition comprenant l'allergène, jusqu'à obtention de la dose efficace. Le patient désensibilisé présente ensuite des réactions allergiques beaucoup plus modérées, voire ne présentent plus de telles réactions, en présence du ou des allergènes en cause. Dans le cas des patients allergiques à l'arachide, la désensibilisation leur permet de consommer des aliments potentiellement en contact avec des arachides, voire des aliments en contenant.

Le sujet à désensibiliser est préférentiellement âgé de 1 an à 18 ans. Les enfants et les adolescents sont particulièrement exposés à l'ingestion accidentelle d'arachide. Par exemple, les enfants ou adolescents peuvent être exposés à l'arachide en mangeant à la cantine, en partageant des friandises avec leurs camarades d'école ou par eux-mêmes car non conscients de la présence potentielle de l'arachide ou de ses traces dans les produits alimentaires. La démarche de désensibilisation présente donc un intérêt tout particulier chez les enfants et les adolescents. Les adolescents allergiques à l'arachide sont reconnus comme particulièrement exposés au risque anaphylactique, et les réactions qu'ils présentent lors d'ingestion accidentelle sont souvent sévères avec un risque létal accentué. Alternativement, le sujet peut être âgé de 18 ans ou plus (de plus de 18 ans).

De manière avantageuse, la gélule selon l'invention est fournie et administrée oralement (ingérée) sous une forme non-ouverte et non-mélangée au bol alimentaire. Le maintien de l'intégrité de la gélule lors de l'ingestion est essentielle présentement en ce qu'elle évite de mettre en contact les cellules immunitaires présentes au niveau de la cavité buccale, du pharynx et de l'œsophage avec l'arachide. Ainsi, l'arachide n'est mise au contact que des cellules immunitaires de l'estomac et de l'intestin grêle. Bien que de nombreux protocoles imposent l'ouverture des gélules et/ou la rupture des enveloppes avant ingestion, pour mélange au bol alimentaire, les inventeurs ont démontré que le shunt du système immunitaire de la cavité buccale, du pharynx et de l'œsophage, et le fait de cibler spécifiquement le système immunitaire de l'intestin grêle, permet d'obtenir une méthode de désensibilisation efficace, tout en minimisant les risques de réactions allergiques.

La méthode d'immunothérapie selon l'invention consiste en une phase d'induction d'une réponse tolérogène du système immunitaire et/ou désensibilisation, suivie d'une phase d'entretien ou maintenance.

La phase de désensibilisation peut consister en l'administration quotidienne par au moins une gélule d'une dose moyenne croissante d'arachide de 10mg à 2g pendant 20 à 28 semaines, préférentiellement pendant 24 semaines. La dose moyenne d'arachide est préférentiellement augmentée jusqu'à un facteur maximum de 2 par palier tous les 10 à 18 jours, préférentiellement tous les 14 jours. Les doses moyennes croissantes d'arachide peuvent être, par exemple : 10mg/jour, 20mg/jour, 40mg/jour, 80mg/jour, 160 mg/jour, 300mg/jour, 500mg/jour, 750mg/jour, 1000mg/jour, 1250mg/jour, 1500mg/jour, et 2000mg/jour.

La phase d'entretien peut consister en l'administration d'une dose moyenne quotidienne d'arachide de l'ordre de 2g, préférentiellement une dose moyenne quotidienne d'arachide comprise entre 2g et 5g. La phase d'entretien est mise en œuvre avec des sujets âgés de 1 à 18 ans, alternativement des sujets de 18 ans ou plus, tolérant une dose cumulée d'arachide supérieure à 2g.

Dans un autre aspect, la présente invention concerne également une gélule à libération gastro-intestinale, telle que définie dans les revendications, destinée à être utilisée par voie orale dans une méthode permettant de diagnostiquer une allergie à l'arachide chez un sujet.

### 5. Liste des figures

D'autres caractéristiques et avantages de l'invention apparaîtront plus clairement à la lecture de la description suivante d'un mode de réalisation préférentiel, donné à titre de simple exemple illustratif et non limitatif, et de la figure annexée présentant un synoptique du déroulement du protocole de l'étude clinique.
La Figure 1 représente un premier diagramme schématisant le déroulement de l'étude.
La Figure 2 représente un second diagramme schématisant le déroulement de l'étude.
Les Figures 3 et 4 représentent l'évolution des rapports IgG/IgE au cours de la phase d'incrémentations

### 6. Description d'un mode de réalisation de l'invention

Le principe général de l'invention repose sur la formulation de gélules comprenant une pâte d'arachide et sur la libération gastro-intestinale de l'arachide (ingestion sous forme non-ouverte), afin de servir d'une part au diagnostic fiable et précis d'une allergie aux arachides et du seuil de déclenchement de l'allergie, et d'autre part à la désensibilisation de l'allergie aux arachides. La formulation sous forme de gélules et leur ingestion sous une forme fermée permet donc d'éviter, ou à tout le moins de fortement réduire, l'effet nocebo pouvant fausser les tests de diagnostic d'une allergie aux arachides. Elle permet également de connaître de manière précise et simple la dose d'arachide administrée au patient. Enfin, elle facilite le protocole de désensibilisation du patient, notamment en évitant tout contact avec la muqueuse buccale riche en cellules immunitaires.

### 6.1. Exemples de compositions de gélules selon l'invention

La pâte d'arachide servant de matière première est composée à 95.7% de cacahuètes, 4.3% d'huile de tournesol ainsi qu'au moins un excipient, à savoir le phosphate tricalcique (excipient pulvérulent), et optionnellement le lactose (excipient prébiotique). La pâte d'arachide est obtenue par mélange de l'arachide avec ladite huile, afin d'obtenir une matière première. Cette pâte d'arachide est ensuite mélangé à des excipients, afin d'obtenir une composition d'arachide selon l'invention. Ainsi, la pâte d'arachide est incorporée sous forme de poudre dans des gélules de différentes tailles.

Les tailles des gélules correspondent à des standards bien précis. L'enveloppe de chaque gélule est composée d'une tête et d'un corps, le corps étant rempli de la pâte d'arachide avant que la tête ne vienne refermer le corps.

Les gélules selon l'invention peuvent prendre toutes les tailles de la taille 000 (la plus grande) à la taille 4 (plus petite).

**Tableau 1 - Détail du standard des tailles de gélules**

| | **000** | **00** | **0** | **1** | **2** | **3** | **4** |
|---|---|---|---|---|---|---|---|
| Longueur gélule fermée (mm) | 26,1 | 23,3 | 21,7 | 19,4 | 18,0 | 15,9 | 14,3 |
| Longueur du corps (mm) | 22,2 | 20,2 | 18,4 | 16,6 | 15,3 | 13 ,6 | 12,2 |
| Longueur de la tête (mm) | 12,9 | 11,7 | 10,7 | 9,8 | 8,9 | 8,1 | 7,2 |
| Diamètre du corps (mm) | 9,5 | 8,2 | 7,3 | 6,6 | 6,1 | 5,6 | 5,0 |
| Diamètre de la tête (mm) | 9,9 | 8,5 | 7,6 | 6,9 | 6,3 | 5,8 | 5,3 |

| | | | | | | | |
|---|---|---|---|---|---|---|---|
| Source : Technical Reference File, 1ère Edition CAPSUGEL- Gélules Coni-Snap | | | | | | | |

On formule selon toutes méthodes bien connues de l'homme de l'art différentes gélules renfermant la pâte d'arachide et un excipient.

**Tableau 2 - Exemples de composition de gélules selon l'invention**

| Dosage (mg) | Pâte d'arachide (mg par gélule) | Phosphate tricalcique (mg par gélule) | Lactose (mg par gélule) | Taille de la gélule |
|---|---|---|---|---|
| 10 | 10.43 | 20.86 | 90 | 4 |
| 20 | 20.86 | 41.72 | 50 | 4 |
| 40 | 41.72 | 41.72 | 20 | 3 |
| 80 | 83.44 | 83.44 | 0 | 3 |
| 160 | 166.88 | 166.88 | 0 | 0 |
| 250 | 260.75 | 260.75 | 100 | 00 |
| 300 | 312.9 | 312.9 | 0 | 00 |
| 500 | 521.5 | 180 | 100 | 00 |

La teneur en protéines de la pâte d'arachide est quantifiée à partir d'échantillons d'arachide brute non grillée. Ceci permet, d'une part, de rendre accessible à l'extraction les protéines de réserve contenues dans la graine et d'autre part, d'éviter les réactions de Maillard nuisibles au dosage. Les extraits protéiques sont ensuite quantifiés par la méthode à l'acide bicinchonininique (méthode Pierce, cf. Smith, P.K. et al., Measurement of protein using bicinchoninic acid. Anal. Biochem. 150 (1985) 76-85). Pour *l'arachis hypogeae* utilisée, la composition en principales fractions allergéniques de la pâte de cacahuète a été analysée et quantifiée par la méthode Elisa. Elle s'élève respectivement à 12%, 15% et 6% pour les taux d'Ara h 1, d'Ara h 3 et d'Ara h 2.

**Tableau 3 - Teneurs en fractions allergéniques Ara h1, Ara h2 et Ara h3.**

| Dosage (mg) | Pâte d'arachide (mg par gélule) | Protéine (mg) | Ara h 1 (mg) | Ara h 3 (mg) | Ara h 2 (mg) | Taille de la gélule |
|---|---|---|---|---|---|---|
| 10 | 10.43 | 2,2 | 0,26 | 0,33 | 0,13 | 4 |
| 20 | 20.86 | 4,38 | 0,53 | 0,66 | 0,26 | 4 |
| 40 | 41.72 | 8,76 | 1,05 | 1,3 | 0,53 | 3 |
| 80 | 83.44 | 17,52 | 2,1 | 2,63 | 1,05 | 3 |
| 160 | 166.88 | 35,04 | 4,2 | 5,26 | 2,1 | 0 |
| 250 | 260.75 | 54,76 | 6,6 | 8,2 | 3,3 | 00 |
| 300 | 312.9 | 65,71 | 7,9 | 9,86 | 3,9 | 00 |
| 500 | 521.5 | 109,52 | 13,1 | 16,4 | 6,6 | 00 |

### 6.2. Essai clinique sur une population adolescente

L'objectif principal de l'étude est d'évaluer l'efficacité d'un protocole de désensibilisation à l'arachide par ingestion de doses croissantes d'arachide jusqu'à un maximum de 2 grammes par jour. La désensibilisation vis-à-vis de l'arachide sera définie par l'absence de symptôme objectif clinique après ingestion d'une dose cumulée de 2 g d'arachide lors d'un Test de Provocation Orale (TPO) en Double Aveugle Contre Placebo (DACP) au terme de la phase d'induction du protocole (TPO2).

### Choix de la population étudiée

Les protocoles d'induction de tolérance à l'arachide se sont intéressés à des populations très hétérogènes d'enfants âgés de 1 à 18 ans. A ce jour, le déposant ne connaît pas d'étude clinique ayant inclus uniquement des adolescents. Or, l'adolescence est une période particulièrement à risque de réaction après ingestion accidentelle. En effet, l'adolescent est moins sujet à guérison spontanée que l'enfant plus jeune. Par ailleurs, l'utilisation de doses fixes d'arachide, dans des protocoles d'induction de tolérance ou des tests diagnostic (TPO), sont plus homogènes en rapport à la surface corporelle.

Le seuil de 500 mg d'arachide détermine l'état de traces pour une quantité de 1000 g d'aliment natif (ftp://ftp.fao.org/docrep/fao/010/y4705f/y4705f02.pdf). La dose de 2 g, soit le quadruple du seuil de « trace », permet de caractériser l'acquisition d'une tolérance aux traces d'arachide. Ainsi, les patients qui tolèrent l'ingestion de 2 g d'arachide sont supposés exempts de risque de réaction avec un régime alimentaire contenant des traces d'arachide.

### Description de la méthodologie de recherche

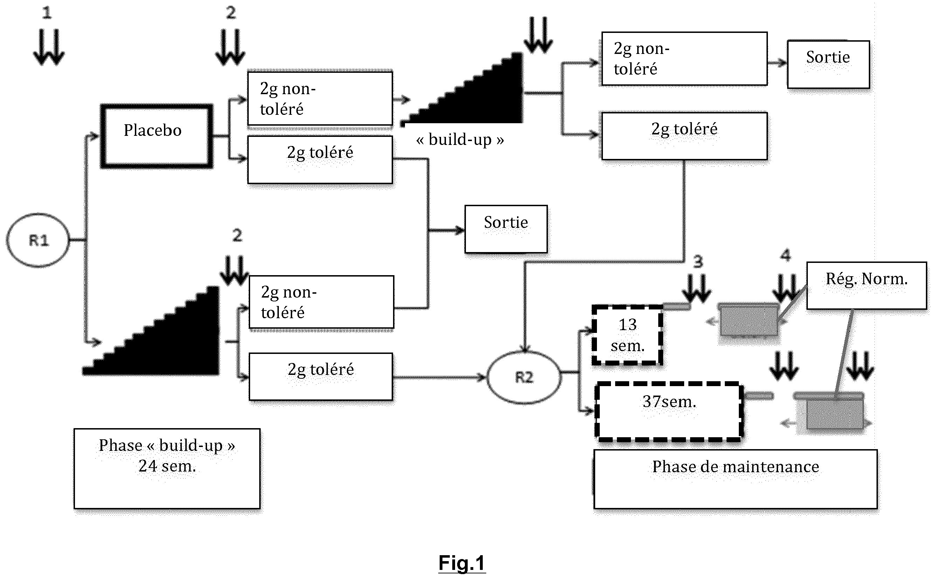

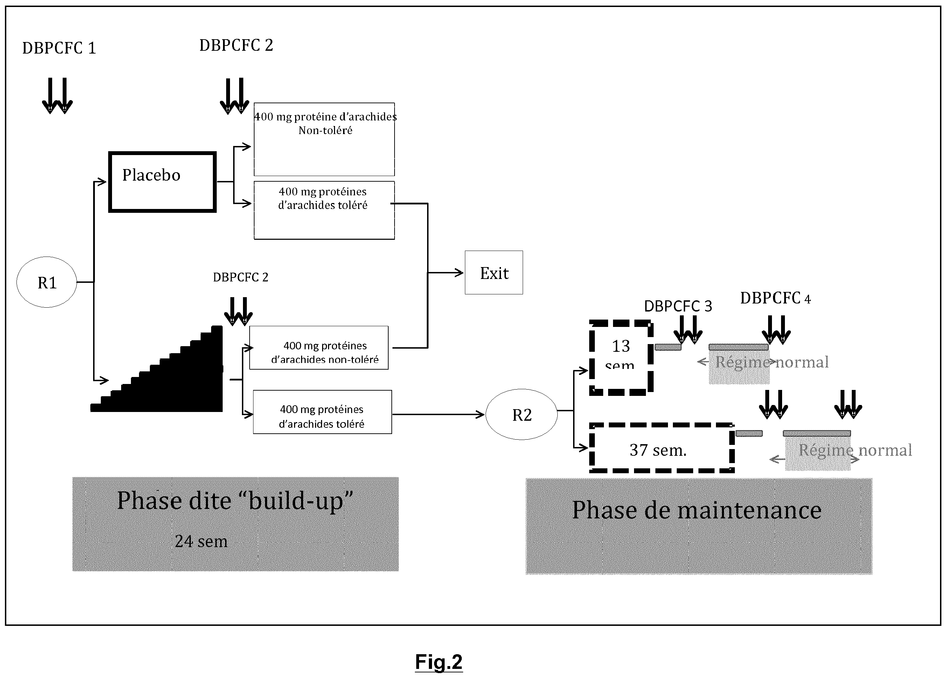

L'étude est multicentrique, randomisée en double aveugle contre placebo et composée de deux phases. Les figures 1 et 2 présentent un synoptique du déroulement du protocole et de la méthodologie employée.

La première phase est une phase de désensibilisation. Les patients reçoivent pendant 24 semaines des doses croissantes soit de placébo, soit de la gélule selon l'invention. Le traitement débute à 10 mg/jour jusqu'à atteindre 2g/ jour, d'arachide ou de placébo, au terme des 24 semaines. L'augmentation de la dose se fait par paliers, tous les 14 jours. En fonction de la survenue et de l'intensité des effets secondaires, il est possible de faire des stagnations (de 14 jours) à la dose induisant les effets secondaires.

Les paliers sont les suivants : 10 mg/jour, 20 mg/jour, 40 mg/jour, 80 mg/jour, 160 mg/jour, 300 mg/jour, 500 mg/jour, 750 mg/jour, 1000 mg/jour, 1250 mg/jour, 1500 mg/jour et 2000 mg/jour.

L'utilisation de la gélule selon l'invention permet le maintien du double aveugle. Ainsi, on attenue chez le patient les effets psychogènes qui pourraient être associés à la prise d'allergène ainsi que l'aversion pour l'arachide native. Par ailleurs le clinicien, qui évalue et grade les effets secondaires, reste objectif dans ses évaluations. Enfin, les cellules immunitaires de la cavité buccale ne sont pas sollicitées.

La seconde phase est une phase d'entretien. Pour être éligibles à la phase d'entretien, les patients doivent tolérer l'ingestion d'une dose cumulée supérieure à 2g d'arachide à la fin de la phase de désensibilisation.

### 6.2.1. Tests de provocation par voie orale (TPO)

Les TPO consistent à ingérer une dose croissante d'arachide, toutes les 30 minutes, jusqu'à 5 grammes. Dans le cadre de ce protocole, tous les TPO seront réalisés en DACP, et permettront de déterminer la dose maximale tolérée. La réaction lors du TPO est définie par la survenue d'une manifestation clinique objective. L'aveugle est levé après la pratique de chaque TPO, ce qui permet ainsi de confirmer ou non le diagnostic d'allergie à l'arachide. Il est pratiqué avant la phase de désensibilisation (TPO1) et au terme de cette période de 24 semaines (TPO2), indépendamment du protocole d'induction de tolérance avec lequel il n'a aucune relation. Les évènements indésirables attendus sont ceux décrits comme conséquence éventuelle d'un test de provocation orale. Ils sont listés dans le tableau 4 ci-dessous.

**Tableau 4 - Effets indésirables et traitements autorisés**

| Signes | Signes subjectifs | Traitement | Signes objectifs | Traitement |
|---|---|---|---|---|
| Locaux | | | Conjonctivite | 2 ± 3 |
| | | | Rhinite; éternuements répétés; obstruction nasale ; rhinorrhée aqueuse | 2 ± 3 |
| | Prurit isolé : labial, oral, voile du palais, pharyngé | 0 ou 3 | Syndrome oral: prurit vélo palatin, œdème des lèvres et dysphagie | 0 ou 3 |
| | Dysphagie | 0 ou 3 | Enanthème | 3 |
| | | | Œdème de la luette | 3 ± 5 |
| Cutanés | Prurit | | | |
| | Isolé | 0 ou 3 | Généralisé ou Palmo-plantaire | 3 |
| | | | Erythème | 0 ou 3 |
| | | | Rash maculo-papuleux | |
| | | | Urticaire | 3 |
| | | | Angio-œdème | 3 ± 5 |
| | | | Eczéma | 1 ± 3 |
| | | | | |
| Digestifs | Douleurs abdominales | | | |
| | Isolées | 0 ou 1 | Répétées ou associées | 0 ou 3 |
| | Nausées | 0 ou 1 | Vomissements | |
| | | | Diarrhée | |
| Respiratoires | | | Modification de la voix | 3 |
| | | | Dyspnée laryngée, stridor | 3 ± 5 |
| | | | Toux, sifflements, dyspnée, crise d'asthme | 4 ± 5 |
| | | | Diminution du VEMS >15% | |
| | | | Diminution du DEP >20% | |
| Généraux | Fatigabilité | 0 ou 1 | Pâleur anormale | 0 |
| | Troubles du comportement | 0 | Accélération du pouls >20% | 0 ou 5 |
| | Céphalées | 0 ou 1 | Diminution de la TA >20 mmHg | |
| | Appréhension, refus de prendre la dose suivante | 0 ou 1 | Diminution de la SaO2 | 4 ± 5 |
| | | | Malaise | 5 ± 6 |
| | | | Choc anaphylactique | |

Les traitements autorisés sont classifiés de la manière suivante:
0. Abstention thérapeutique
1. Traitement symptomatique : émollients ou dermocorticoïdes, antiémétiques, anti spasmodiques, paracétamol
2. Antihistaminiques par voie locale
3. Antihistaminiques par voie générale
4. Beta deux mimétiques inhalés
5. Glucocorticoïdes systémiques
6. Adrénaline

L'anaphylaxie est définie comme l'apparition rapide (quelques minutes à quelques heures), après exposition à un probable allergène, de symptômes témoignant d'une atteinte de 2 organes (Sampson et al. J. All. Clin. Immunol., Février 2006; 117(2):391-397). A titre d'exemple, les symptômes dermatologiques en cause sont le prurit ou rash généralisé, l'œdème des lèvres, de la langue ou de la luette. Les symptômes respiratoires sont la dyspnée, les sifflements, la toux laryngée, la diminution du débit expiratoire de pointe et l'hypoxémie. Les symptômes digestifs sont les crampes abdominales, les vomissements et la diarrhée. Les symptômes généraux sont la chute de la tension artérielle ou l'association de symptômes de défaillance d'organe : hypotonie, perte de connaissance, incontinence...

Ces symptômes sont classés par stade de gravité selon la classification de Ring et Messmer modifiée (The diagnosis and management of anaphylaxis: an updated practice parameter. J. Allergy Clin. Immunol. 2005; 115:S483-523) :
- Grade 1 : conjonctivite, rhinite, syndrome d'allergie orale, urticaire généralisée simple, œdème des lèvres et/ou du visage sans symptômes d'asphyxie (gêne respiratoire), toux ou sifflements isolés, nausées ou douleurs abdominales ;
- Grade 2 : Atteinte multiviscérale modérée avec signes cutanéo-muqueux (angio-oedeme) ± signes digestifs (vomissements, diarrhée) ± asthme (bronchospasme aigu) : toux, dyspnée, sifflements, chute du débit expiratoire de pointe (15 % ou plus des valeurs, attendues ou connues) ± fatigabilité et tachycardie ;
- Grade 3 : Atteinte multiviscérale sévère menaçant la vie et imposant une thérapeutique spécifique : bronchospasme ou signes d'œdème laryngé avec signes d'asphyxie, anaphylaxie (symptômes d'atteinte de plusieurs organes, incluant des symptômes respiratoires) et choc anaphylactique (malaise, agitation, perte de connaissance, collapsus), collapsus cardiovasculaire, tachycardie ou bradycardie, troubles du rythme cardiaque ; et
- Grade 4 : arrêt cardiorespiratoire.

L'analyse statistique est réalisée sous STATA12 (StataCorp, College Station, TX) et R (http://cran.r-project.org/).

### 6.2.2. Résultats

Parmi les 53 patients testés, 30 patients ont été inclus car ils présentaient un seuil de développement de l'allergie compris entre 100 mg et 2000 mg d'arachide. Au début de l'étude, aucune différence statistique significative n'apparaît entre le groupe test et le groupe témoin en termes d'âge, de sexe, d'indice de masse corporelle, de mesure des dosages d'IgE totales et spécifiques, d'IgG4 totales et spécifiques, de type de réaction observée au diagnostic ou de nombre de survenue de réaction sévère après diagnostic, de pathologies respiratoires ou associés à l'alimentation ni même de passé médical.

### Efficacité clinique

Les résultats d'efficacité clinique sont présentés au tableau 5. Parmi les 30 patients inclus, 28 patients ont suivis la seconde phase du test de provocation orale dont 19 ont été exposé à l'arachide et 9 au placébo. 17 des 21 patients du groupe arachide sont parvenus au seuil de tolérance de 2 g cumulés d'arachide. Ce seuil n'a été atteint que pour 1 patient sur 9 avec le placébo (p<0,001). On observe une augmentation significative du seuil de positivité pendant le TPO chez les patients du groupe traité (p < 0.001). Ce seuil est augmenté de 15 fois dans le groupe traité et de 3 fois dans le groupe placebo. De plus, le nombre de patients ayant quadruplé leur seuil de tolérance entre la première et la seconde phase du TPO est également significativement supérieur chez les patients du groupe arachide par rapport aux patients du groupe placebo (p < 0.001).

**Tableau 5 - Efficacité clinique**

| | | Arachide (n = 19) | Placébo (n=9) | p |
|---|---|---|---|---|
| TPO2 à 2 grammes | Négatif * | 17 (89%) | 1 (11%) | <0.001 |
| | Positif ** | 2 (11%) | 8 (89%) | |
| Seuil de réactivité | TPO1 m; [Q25 Q75] | 610 [610-2000] | 610 [310-1100] | 0.38 |
| | TPO2 m; [Q25 Q75] | 9000 [4000-9000] | 2000 [1100-2000] | <0.001 |
| | *p (TPO2*/*TPO1)* | *p <0.001* | *p =0.02* | |
| | X4 | 17 | 2 | < 0.001 |

| | | | | |
|---|---|---|---|---|
| Légende : * = patients ne tolérant pas 2 grammes cumulés d'arachide. ** = patients tolérant 2 grammes cumulés d'arachide. m = moyenne Q25 = 1^{er} quartile Q75 = 3^{ème} quartile | | | | |

### Tolérance au traitement

Les résultats sur la tolérance sont compilés dans le tableau 6. Comme on peut le constater, 2 patients n'ont pas réussi à terminer la première phase d'induction. Le premier patient a dû abandonner en raisons d'effets indésirables modérés qui l'ont obligé à recommencer plus de 3 fois un palier de 14 jours, avec la même dose d'arachide. Le second patient a fait un choc anaphylactique 2 heures après la prise de la gélule d'arachide, ce qui a nécessité l'administration d'épinéphrine et d'une prise en charge aux urgences. A posteriori, le patient a reconnu avoir pris deux fois la dose d'arachide prescrite. Aucune réaction ou effets secondaires sérieux n'a été observé dans le groupe témoin.

Des effets indésirables modérés ont été observés chez le même nombre de patients : 19/21 chez les patients du groupe arachide contre 8/9 chez les patients du groupe témoin. Toutefois, les patients appartenant au groupe arachide ont fait plus d'effets indésirables que ceux du groupe témoin : 6,19 +/-3,17 réactions pour le groupe arachide v. 3,66+/-2,29 réactions pour le groupe témoin (p<0,05).

De même, le nombre de patients qui ont terminé un palier de 14 jours sans présenter d'effet indésirable était significativement supérieur dans le groupe témoin que dans le groupe arachide (p=0.001). De plus, des différences significatives ont également été observées dans l'intensité des effets indésirables, selon la classification de Ring et Messmer. Les patients du groupe témoin ont développé davantage d'effets indésirables du grade 1 alors que les patients du groupe arachide ont davantage connu d'effets indésirables relevant des grades 2 et 3 (p< 0,05).

En ce qui concerne le recours aux soins d'urgence, aucune différence significative n'a été observée entre les deux groupes. Les patients du groupe arachide ont toutefois ressenti significativement plus de troubles digestifs (p<0,01) et respiratoires (p=0,01) que les patients du groupe témoin (68 v. 7, et 61 v. 7 respectivement). Les effets indésirables oropharyngés et la dysphagie sont rares et ne sont pas plus fréquents dans le groupe traité que dans le groupe placebo (tableau 6b). Aucune œsophagite à éosinophiles n'a été suspectée dans le groupe traité et aucun patient n'a été exclu du fait de symptômes gastro-intestinaux. Les réactions générales sont également réparties entre le groupe placebo et le groupe traité. Les réactions multi-systèmes sont rares dans le groupe traité (une seule classée en SAE severe adverse event chez les 19 patients du groupe arachide) et surviennent au minimum 40 min après l'ingestion de la gélule.

**Tableau 6a - Effets secondaires de la phase d'induction de la tolérance à l'arachide**

| | | Groupe arachide | Groupe placébo | p |
|---|---|---|---|---|
| N = | | 21 | 9 | |
| Effets indésirables (E.I.) sévères | | 1 | 0 | n.s. |
| Exclusion du protocole | | 2 | 0 | n.s. |
| Nombre de patients avec des E.I. | Non | 2 | 1 | n.s. |
| | ≥ 1 | 19 | 8 | |
| Nombre de réaction/ patient | | 6.19±3.17 | 3.66±2.29 | < 0.05 |
| Nombre de palier de 2 semaines | Sans E.I. | 162/292 | 88/121 | 0.001 |
| | Avec E.I. | 130/292 | 33/121 | |
| Intensité de l'E.I.* (n=) | 1 | 93 | 30 | <0.05 |
| | 2 | 32 | 2 | |
| | 3 | 5 | 0 | |
| | 4 | 0 | 0 | n.s. |
| Nombre d'E.I. | Local | 30 | 8 | n.s. |
| | Cutané | 48 | 16 | n.s. |
| | Digestif | 68 | 7 | <0.01 |
| | Respiratoire | 61 | 7 | 0.01 |
| | Général | 18 | 2 | n.s. |
| Traitement des E.I. | oui | 88 | 21 | n.s |
| | non | 42 | 12 | |
| Hospitalisation pour E.I. | | 1 | 0 | n.s |

**Tableau 6b - Effets indésirables (EI) locaux pendant la phase d'incrémentations : nombre de réactions locales entre parenthèses le pourcentage de patients**

| EI local | Arachide (n =21) | placebo (n=9) | p = |
|---|---|---|---|
| Conjonctivites | 10 (24) | 2 (22) | 0.96 |
| Rhinites | 21 (43) | 5 (33) | 0.77 |
| Symptômes oropharyngés | 6 (19) | 1 (11) | 0.71 |
| Dysphagie | 0 (0) | 0 (0) | ns |

### Désensibilisation et modifications immunologiques (tableau 7)

Les résultats de la désensibilisation démontrent que les patients du groupe traités par la gélule selon l'invention (groupe Ara) modifient significativement leur bilan immunitaire vis-à-vis de l'arachide à la différence des patients traités par placebo (groupe Plac). Les tests dermatologiques cutanés révèlent une diminution du diamètre de la réaction allergique passant de 12,45 ± 6,71 mm à 6,05 ± 4,06 mm (p < 0,0001) entre les deux phases du TPO. Des changements moins significatifs ont été observés dans le groupe témoin (12,5 ± 6.7mm to 8 ± 3,7mm; p<0,05).

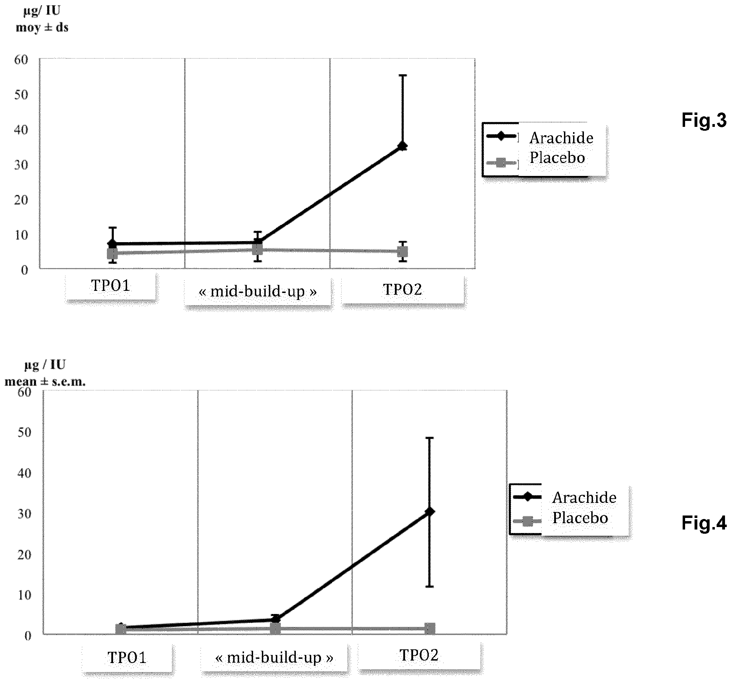

Dans le groupe arachide (Ara), le niveau des dosages sériques d'IgE spécifiques à l'arachide augmente de façon significative tout au long de cette phase d'induction de tolérance (p=0,001). La même cinétique a été observée avec les IgE spécifiques de la fraction allergénique Ara h2. Des modifications significatives quant à la réactivité envers les allergènes Ara h1 et Ara h2 ont été observées chez les patients du groupe arachide (p=0.01). En particulier, les dosages d'IgE spécifiques d'Ara h1 n'ont atteint des niveaux significatifs qu'en seconde phase. En revanche, les dosages d'IgE spécifiques pour Ara h3, Ara h8 et Ara h9 n'ont pas évolué de manière significative chez les patients du groupe arachide, par rapport à leur niveau de départ, et n'ont pas du tout été modifié chez les patients du groupe témoin. Chez les patients du groupe placébo (Plac), aucune modification significative n'a été observée pour les IgE spécifiques de l'arachide ainsi que de ses différentes fractions allergéniques. A l'inverse les IgG4 spécifiques de l'arachide augmentent de façon significative dans le groupe traité mais pas dans le groupe placebo. Il en est de même pour, les trois principales fractions protéiques Ara h1, Ara h2 et Ara h3. Le rapport des anticorps spécifiques IgG4 / IgE augmente de façon importante entre le début et la fin de la phase d'incrémentation pour le groupe arachide mais reste stable pour le groupe témoin. L'augmentation de ce rapport qui reflète indirectement l'apparition d'une désensibilisation est significative pour la fraction ara h2 (p = 0.03). (figures 3 et 4)

**Tableau 7 - Effets de la désensibilisation sur le bilan immunologique**

| | | TPO 1 | | Milieu phase d'induction | TPO 2 | | |
|---|---|---|---|---|---|---|---|
| | | m±sd | p | m±sd (p*) | m±sd (p**) | p * * | p |
| diamètre de la réaction (mm) | Ara. | 12.4±6.7 | ns | | 6.1±4 (<0.0001) | | 0.58 |
| | Plac. | 12.5±6.5 | | | 8±3.8 (<0.05) | | |
| Total IgE (IU/mL) | Ara | 628±469 | ns | 698±411 (0.357) | 840±573 (0.004) | | 0.014 |
| | Plac | 1266±1504 | | 1193±1511 (0.324) | 1175±1591 (0.264) | | |
| Peanut Sp. IgE (IU/mL) | Ara | 62.5±34.5 | ns | 76.0±34.7 (0.001) | 78.6±32.8 (<0.001) | | 0.044 |
| | Plac | 79.0±30.7 | | 78.7±32.9 (0.880) | 79.3±32.1 (0.244) | | |
| Ara h1 Sp IgE (IU/mL) | Ara | 33.4±36.6 | ns | 38.1±39.1 (0.227) | 41.6±38.1 (0.011) | | 0.10 |
| | Plac | 33.6±29.0 | | 31.3±22.0 (0.421) | 28.95±25.0 (0.924) | | |
| Ara h2 Sp IgE (IU/mL) | Ara | 50.4±35.9 | ns | 63.0±35.6 (0.009) | 62.5±35.0 (0.010) | | 0.25 |
| | Plac | 70.9±34.2 | | 73.44±36.03 (0.276) | 71.38±36.85 (0.105) | | |
| Ara h3 Sp IgE (IU/mL) | Ara | 24.8±38.5 | ns | 32.43±41.69 (0.160) | 31.12±38.31 (0.084) | | 0.20 |
| | Plac | 23.9±32.9 | | 23.7±31.9 (0.881) | 21.3±31.0 (0.528) | | |
| Ara h8 Sp IgE (IU/mL) | Ara | 1.5±5.2 | <0. 01 | 1.2±3.5 (0.451) | 2.2±5.3 (0.272) | | 0.06 |
| | Plac | 17.6±32.6 | | 19.8±33.7 (0.421) | 24.3±33.2 (0.126) | | |
| Ara h9 Sp IgE (IU/mL) | Ara | 0.2±0.7 | ns | 0.1±0.4 (0.743) | 0.2±0.7 (0.650) | | 0.38 |
| | Plac | 1.0±1.6 | | 0.9±1.5 (0.826) | 1.5±2.8 (0.403) | | |
| Total IgG4 (mg/mL) | Ara | 0.86±0.84 | ns | 0.85±0.80 (0.217) | 0.93±0.82 (0.240) | | 0.36 |
| | Plac | 0.83±0.63 | | 0.88±0.73 (0.429) | 0.78±0.59 (0.348) | | |
| Peanut sp IgG4 (mg/mL) | Ara | 0.40±0.45 | ns | 0.99±0.85 (0.303) | 2.98±3.69 (<0.001) | | 0.04 |
| | Plac | 0.44±0.53 | | 0.50±0.53 (0.458) | 0.42±0.44 (0.797) | | |
| Ara h1 sp IgG4 (mg/mL) | Ara | 0.04±0.05 | ns | 0.06±0.07 (0.909) | 0.38±0.89 (0.026) | | 0.12 |
| | Plac | 0.036±0.04 | | 0.04±0.05 (0.078) | 0.02±0.02 (0.851) | | |
| Ara h2 sp IgG4 (mg/mL) | Ara | 0.09±0.10 | ns | 0.31±0.46 (0.627) | 1.46±2.87 (0.003) | | 0.49 |
| | Plac | 0.08±0.05 | | 0.10±0.08 (0.002) | 0.07±0.06 (0.490) | | |
| Ara h3 sp IgG4 (mg/mL) | Ara | 0.22±0.40 | ns | 0.24±0.33 (0.787) | 0.55±0.53 (0.0001) | | 0.006 |
| | Plac | 0.08±0.07 | | 0.12±0.10 (0.004) | 0.09±0.10 (0.288) | | |
| Ara h8 sp IgG4 (mg/mL) | Ara | 0.01±0.03 | ns | 0.01±0.04 (0.778) | 0.01±0.058 (0.582) | | 0.029 |
| | Plac | 0.11±0.18 | | 0.11±0.15 (0.897) | 0.17±0.24 (0.108) | | |
| Ara h9 sp IgG4 (mg/mL) | Ara | 0.04±0.13 | ns | 0.06±0.21 (0.849) | 0.20±0.77 (0.146) | | 0.17 |
| | Plac | 0.34±0.79 | | 0.26±0.55 (0.218) | 0.3±0.57 (0.275) | | |

| | | | | | | | |
|---|---|---|---|---|---|---|---|
| **Légende:** m± sd = moyenne ± écart-type Ara = groupe arachide ou groupe traité par la gélule selon l'invention Plac = groupe placébo. sp = spécifique | | | | | | | |

Les figures 3 et 4 représentent l'évolution des rapports IgG/IgE au cours de la phase d'incrémentations. Dans la figure 3 (rapport IgG4 / IgE spécifiques de l'arachide), on observe une Augmentation (p=0.06) du rapport des anticorps IgG4 (µg/mL)/ IgE (IU/mL) spécifiques de l'arachide entre TPO1 et TPO2. Dans la figure 4, on observe une augmentation significative (p=0.03) du rapport des anticorps IgG4 (µg/mL)/ IgE (IU/mL) spécifiques de la fraction ara h2 de l'arachide entre TPO1 et TPO2.

### 7. Conclusion

La gélule d'arachide et son ingestion sous forme fermée (non-ouverte) selon l'invention permet d'éviter le contact de la composition d'arachide qu'elle contient avec la muqueuse buccale pour différentes raisons.

La première raison est que la muqueuse buccale est riche en cellules présentatrices de l'antigène, ce qui expose au risque de réaction allergique, voire de choc anaphylactique et limite ainsi les possibilités de désensibilisation. A l'inverse, les inventeurs proposent d'exposer l'allergène directement au contact des cellules présentatrices d'antigène de l'intestin grêle, siège physiologique de l'induction de la tolérance orale aux antigènes alimentaires, afin de réduire les risques et l'intensité des réactions allergiques pendant la désensibilisation tout en maintenant l'efficacité thérapeutique.

La seconde raison est que la muqueuse buccale est le siège du goût et en partie de l'odorat. Or, l'arachide présente une odeur et une saveur bien caractéristiques et difficiles à masquer. L'administration par gélule avec une libération intestinale permet d'éviter l'effet nocébo, à savoir qu'un patient supposant être en train de consommer la substance allergisante, déclenche une réaction allergique.

Les résultats de l'essai clinique en double aveugle démontrent que l'administration de la gélule selon l'invention permet d'une part de pratiquer de façon rigoureuse la mesure du seuil de réactivité à l'allergène par la pratique d'un test de provocation en double aveugle contre placebo, et d'autre part de désensibiliser efficacement les patients traités, tout en réduisant le risque de survenue de réaction allergique chez eux, particulièrement le risque d'œsophagite à éosinophiles et de choc anaphylactique.

## Revendications

1. Gélule à libération gastro-intestinale pour utilisation par voie orale dans une méthode permettant de désensibiliser et/ou d'induire une tolérance chez un sujet allergique à l'arachide,
ladite gélule comprenant une enveloppe et un cœur, ledit cœur comprenant une composition d'arachides comprenant de l'arachide, au moins une huile, au moins un premier excipient pulvérulent, et optionnellement au moins un second excipent prébiotique ;
ladite gélule étant fournie et ingérée sous une forme non-ouverte ; préférentiellement ladite gélule étant fournie sous une forme non-ouverte et non-mélangée au bol alimentaire.

2. Gélule pour utilisation selon la revendication 1, **caractérisée en ce que** les arachides sont des arachides grillées, préférentiellement des arachides grillées et non-dégraissées.

3. Gélule pour utilisation selon l'une quelconque des revendications précédentes, **caractérisée en ce que** ladite composition comprend entre 10 mg et 1000 mg d'arachide, préférentiellement entre 10 mg et 750 mg d'arachide, très préférentiellement entre 10 mg et 500 mg d'arachide.

4. Gélule pour utilisation selon l'une quelconque des revendications précédentes, **caractérisée en ce que** ladite composition comprend entre 5% et 70% d'arachide, par rapport au poids total de ladite composition.

5. Gélule pour utilisation selon l'une quelconque des revendications précédentes, **caractérisée en ce que** ladite composition comprend entre 10% et 40%, préférentiellement entre 10% et 30%, très préférentiellement entre 15% et 25%, de protéines allergéniques, par rapport au poids total de ladite composition.

6. Gélule pour utilisation selon l'une quelconque des revendications précédentes, **caractérisée en ce que** ladite composition comprend entre 10% et 15% des protéines totales pour la fraction allergénique Ara h1, entre 2% et 10% des protéines totales pour la fraction allergénique Ara h2, et entre 10% et 20% des protéines totales pour la fraction allergénique ARA h3, par rapport au poids total de protéines de ladite composition.

7. Gélule pour utilisation selon l'une quelconque des revendications précédentes, **caractérisée en ce que** ladite huile est de l'huile de tournesol.

8. Gélule pour son utilisation selon l'une quelconque des revendications précédentes, **caractérisée en ce que** ledit premier excipient pulvérulent est le phosphate tricalcique ; préférentiellement le premier excipient pulvérulent est choisi parmi le phosphate tricalcique bêta, le phosphate tricalcique alpha, et leur mélange; alternativement le premier excipient pulvérulent est le phosphate tricalcique bêta ou le phosphate tricalcique alpha.

9. Gélule pour utilisation selon l'une quelconque des revendications précédentes, **caractérisée en ce que** ledit second excipient prébiotique est le lactose, préférentiellement le lactose monohydraté.

10. Gélule pour utilisation selon l'une quelconque des revendications précédentes, **caractérisée en ce que** ladite composition comprend entre 20 mg et 315 mg du premier excipient pulvérulent, et/ou entre 0 mg et 100 mg du second excipient prébiotique.

11. Gélule pour utilisation selon l'une quelconque des revendications précédentes, **caractérisée en ce que** ladite composition comprend entre 15% et 55% du premier excipient pulvérulent, par rapport au poids total de ladite composition.

12. Gélule pour utilisation selon l'une quelconque des revendications précédentes, **caractérisée en ce que** ladite composition comprend entre 10% et 75% du second excipient prébiotique, par rapport au poids total de ladite composition.

13. Gélule pour utilisation selon l'une quelconque des revendications précédentes, **caractérisée en ce que** le sujet est âgé de 1 à 18 ans, ou alternativement de 18 ans ou plus.

14. Gélule pour utilisation selon l'une quelconque des revendications précédentes, **caractérisée en ce que** la méthode d'immunothérapie consiste en une phase désensibilisation et/ou d'induction d'une réponse tolérogène du système immunitaire, suivie d'une phase d'entretien et/ou de maintenance de la tolérance.

15. Gélule pour utilisation selon la revendication 14, **caractérisée en ce que** la phase de désensibilisation et/ou d'induction d'une réponse tolérogène consiste en l'administration quotidienne par au moins une gélule d'une dose moyenne croissante d'arachide de 10 mg à 2 g pendant 20 à 28 semaines, préférentiellement pendant 24 semaines.

16. Gélule pour utilisation selon l'une des revendications 14 et 15, **caractérisée en ce que** la phase d'entretien et/ou de maintenance de tolérance consiste en l'administration d'une dose moyenne quotidienne d'arachide supérieure ou égale à 2g, préférentiellement une dose moyenne quotidienne d'arachide comprise entre 2g et 5g ; préférentiellement **en ce que** la phase d'entretien et/ou de maintenance de tolérance est mise en œuvre avec des sujets âgés de 1 à 18 ans tolérant une dose cumulée d'arachide supérieure à 2g.

17. Gélule à libération gastro-intestinale pour utilisation par voie orale dans une méthode permettant de diagnostiquer une allergie à l'arachide chez un sujet, ladite gélule comprenant une enveloppe et un cœur, ledit cœur comprenant une composition d'arachides comprenant de l'arachide, au moins une huile, au moins un premier excipient pulvérulent, et au moins un second excipent prébiotique ;
ladite gélule étant fournie et ingérée sous une forme non-ouverte ; préférentiellement ladite gélule étant fournie sous une forme non-ouverte et non-mélangée au bol alimentaire.

## Patentansprüche

1. Kapsel zur gastrointestinalen Freisetzung für die orale Verwendung in einem Verfahren, das eine Desensibilisierung und/oder Verträglichkeitsinduzierung bei einem Patienten mit Erdnussallergie ermöglicht,
wobei die Kapsel eine Hülle und einen Kern umfasst, wobei der Kern eine Erdnusszusammensetzung, umfassend Erdnüsse, mindestens ein Öl, mindestens einen ersten pulverförmigen Hilfsstoff und optional mindestens einen zweiten präbiotischen Hilfsstoff, umfasst;
wobei die Kapsel in einer ungeöffneten Form bereitgestellt und eingenommen wird; wobei die Kapsel vorzugsweise in einer ungeöffneten und nicht mit dem Speisebrei vermischten Form bereitgestellt wird.

2. Kapsel zur Verwendung gemäß Anspruch 1, **dadurch gekennzeichnet, dass** die Erdnüsse geröstete Erdnüsse sind, vorzugsweise geröstete und nicht entfettete Erdnüsse.

3. Kapsel zur Verwendung gemäß einem der vorhergehenden Ansprüche, **dadurch gekennzeichnet, dass** die Zusammensetzung zwischen 10 mg und 1000 mg Erdnuss, vorzugsweise zwischen 10 mg und 750 mg Erdnuss, besonders bevorzugt zwischen 10 mg und 500 mg Erdnuss, umfasst.

4. Kapsel zur Verwendung gemäß einem der vorhergehenden Ansprüche, **dadurch gekennzeichnet, dass** die Zusammensetzung zwischen 5 % und 70 % Erdnuss umfasst, bezogen auf das Gesamtgewicht der Zusammensetzung.

5. Kapsel zur Verwendung gemäß einem der vorhergehenden Ansprüche, **dadurch gekennzeichnet, dass** die Zusammensetzung zwischen 10 % und 40 %, vorzugsweise zwischen 10 % und 30 %, besonders bevorzugt zwischen 15 % und 25 % allergene Proteine umfasst, bezogen auf das Gesamtgewicht der Zusammensetzung.

6. Kapsel zur Verwendung gemäß einem der vorhergehenden Ansprüche, **dadurch gekennzeichnet, dass** die Zusammensetzung zwischen 10 % und 15 % Gesamtproteine für die allergene Fraktion Ara h1, zwischen 2 % und 10 % Gesamtproteine für die allergene Fraktion Ara h2 und zwischen 10 % und 20 % Gesamtproteine für die allergene Fraktion Ara h3 bezogen auf das Gesamtproteingewicht der Zusammensetzung umfasst.

7. Kapsel zur Verwendung gemäß einem der vorhergehenden Ansprüche, **dadurch gekennzeichnet, dass** das Öl Sonnenblumenöl ist.

8. Kapsel zu ihrer Verwendung gemäß einem der vorhergehenden Ansprüche, **dadurch gekennzeichnet, dass** der erste pulverförmige Hilfsstoff Trikalziumphosphat ist; dass vorzugsweise der erste pulverförmige Hilfsstoff aus Beta-Trikalziumphosphat, Alpha-Trikalziumphosphat und einem Gemisch daraus ausgewählt ist; oder dass alternativ der erste pulverförmige Hilfsstoff Beta-Trikalziumphosphat oder Alpha-Trikalziumphosphat ist.

9. Kapsel zur Verwendung gemäß einem der vorhergehenden Ansprüche, **dadurch gekennzeichnet, dass** der zweite präbiotische Hilfsstoff Laktose, vorzugsweise Laktosemonohydrat, ist.

10. Kapsel zur Verwendung gemäß einem der vorhergehenden Ansprüche, **dadurch gekennzeichnet, dass** die Zusammensetzung zwischen 20 mg und 315 mg des ersten pulverförmigen Hilfsstoffes und/oder zwischen 0 mg und 100 mg des zweiten präbiotischen Hilfsstoffes umfasst.

11. Kapsel zur Verwendung gemäß einem der vorhergehenden Ansprüche, **dadurch gekennzeichnet, dass** die Zusammensetzung zwischen 15 % und 55 % des ersten pulverförmigen Hilfsstoffes umfasst, bezogen auf das Gesamtgewicht der Zusammensetzung.

12. Kapsel zur Verwendung gemäß einem der vorhergehenden Ansprüche, **dadurch gekennzeichnet, dass** die Zusammensetzung zwischen 10 % und 75 % des zweiten präbiotischen Hilfsstoffes umfasst, bezogen auf das Gesamtgewicht der Zusammensetzung.

13. Kapsel zur Verwendung gemäß einem der vorhergehenden Ansprüche, **dadurch gekennzeichnet, dass** der Patient im Alter von 1 bis 18 Jahren ist, oder alternativ von 18 Jahren oder mehr.

14. Kapsel zur Verwendung gemäß einem der vorhergehenden Ansprüche, **dadurch gekennzeichnet, dass** das Immuntherapieverfahren aus einer Phase der Desensibilisierung und/oder Induzierung einer tolerogenen Antwort des Immunsystems, gefolgt von einer Phase der Erhaltung und/oder Weiterentwicklung der Toleranz besteht.

15. Kapsel zur Verwendung gemäß Anspruch 14, **dadurch gekennzeichnet, dass** die Phase der Desensibilisierung und/oder Induktion einer tolerogenen Antwort aus der täglichen Verabreichung, durch mindestens eine Kapsel, einer steigenden durchschnittlichen Erdnussdosis von 10 mg bis 2 g während 20 bis 28 Wochen, vorzugsweise während 24 Wochen, besteht.

16. Kapsel zur Verwendung gemäß einem der Ansprüche 14 und 15, **dadurch gekennzeichnet, dass** die Phase der Erhaltung und/oder Weiterentwicklung der Toleranz in der Verabreichung einer täglichen durchschnittlichen Erdnussdosis größer oder gleich 2 g, vorzugsweise einer täglichen durchschnittlichen Erdnussdosis zwischen 2 g und 5 g besteht; dass vorzugsweise die Phase der Erhaltung und/oder Weiterentwicklung der Toleranz an Patienten im Alter von 1 bis 18 Jahren eingesetzt wird, die eine kumulierte Erdnussdosis von über 2 g tolerieren.

17. Kapsel zur gastrointestinalen Freisetzung für die orale Verwendung in einem Verfahren, das es ermöglicht, eine Erdnussallergie bei einem Patienten zu diagnostizieren, wobei die Kapsel eine Hülle und einen Kern umfasst, wobei der Kern eine Erdnusszusammensetzung umfasst, umfassend Erdnuss, mindestens ein Öl, mindestens einen ersten pulverförmigen Hilfsstoff und mindestens einen zweiten präbiotischen Hilfsstoff;
wobei die Kapsel in einer ungeöffneten Form bereitgestellt und eingenommen wird, wobei die Kapsel vorzugsweise in einer ungeöffneten und nicht mit dem Speisebrei vermischten Form bereitgestellt wird.

## Claims

1. A gastro-intestinal release capsule intended for oral use in a method which enables desensitisation and/or a tolerance to be induced in a subject allergic to peanuts,
the capsule comprising a shell and a core, the core comprising a peanut composition which comprises peanut, at least one oil, at least a first pulverulent excipient and optionally at least a second prebiotic excipient;
the capsule being provided and ingested in a non-open form; the capsule preferably being provided in a form which is non-open and not mixed with the bolus.

2. Capsule for use according to claim 1, **characterised in that** the peanuts are roasted peanuts, preferably roasted and non-defatted peanuts.

3. Capsule for use according to either of the preceding claims, **characterised in that** the composition comprises between 10 mg and 1000 mg of peanut, preferably between 10 mg and 750 mg of peanut, very preferably between 10 mg and 500 mg of peanut.

4. Capsule for use according to any one of the preceding claims, **characterised in that** the composition comprises between 5% and 70% of peanut in relation to the total weight of the composition.

5. Capsule for use according to any one of the preceding claims, **characterised in that** the composition comprises between 10% and 40%, preferably between 10% and 30%, very preferably between 15% and 25%, of allergenic proteins in relation to the total weight of the composition.

6. Capsule for use according to any one of the preceding claims, **characterised in that** the composition comprises between 10% and 15% of the total proteins for the allergenic fraction Ara h1, between 2% and 10% of the total proteins for the allergenic fraction Ara h2 and between 10% and 20% of the total proteins for the allergenic fraction ARA h3 in relation to the total weight of proteins of the composition.

7. Capsule for use according to any one of the preceding claims, **characterised in that** the oil is sunflower oil.

8. Capsule for use according to any one of the preceding claims, **characterised in that** the first pulverulent excipient is tricalcium phosphate; preferably, the first pulverulent excipient is selected from beta tricalcium phosphate, alpha tricalcium phosphate, and the admixture thereof; alternatively, the first pulverulent excipient is beta tricalcium phosphate or alpha tricalcium phosphate.

9. Capsule for use according to any one of the preceding claims, **characterised in that** the second prebiotic excipient is lactose, preferably lactose monohydrate.

10. Capsule for use according to any one of the preceding claims, **characterised in that** the composition comprises between 20 mg and 315 mg of the first pulverulent excipient and/or between 0 mg and 100 mg of the second prebiotic excipient.

11. Capsule for use according to any one of the preceding claims, **characterised in that** the composition comprises between 15% and 55% of the first pulverulent excipient in relation to the total weight of the composition.

12. Capsule for use according to any one of the preceding claims, **characterised in that** the composition comprises between 10% and 75% of the second prebiotic excipient in relation to the total weight of the composition.

13. Capsule for use according to any one of the preceding claims, **characterised in that** the subject is aged from 1 to 18 years, or alternatively 18 years or above.

14. Capsule for use according to any one of the preceding claims, **characterised in that** the immunotherapy method involves a phase for desensitisation and/or inducing a tolerogenic response of the immune system followed by a phase for retaining and/or maintaining the tolerance.

15. Capsule for use according to claim 14, **characterised in that** the phase for desensitisation and/or inducing a tolerogenic response involves daily administration via at least one capsule of an increasing average dose of peanut of from 10 mg to 2 g for from 20 to 28 weeks, preferably for 24 weeks.

16. Capsule for use according to either claim 14 or 15, **characterised in that** the phase for retaining and/or maintaining the tolerance involves the administration of a mean daily dose of peanut greater than or equal to 2 g, preferably a mean daily dose of peanut between 2 g and 5 g; preferably **in that** the phase for retaining and/or maintaining the tolerance is implemented with subjects aged from 1 to 18 years tolerating a cumulative dose of peanut greater than 2 g.

17. A gastro-intestinal release capsule intended for oral use in a method which enables a peanut allergy to be diagnosed in a subject, the capsule comprising a shell and a core, the core comprising a peanut composition comprising peanut, at least one oil, at least a first pulverulent excipient and at least a second prebiotic excipient;
the capsule being provided and ingested in a non-open form; the capsule preferably being provided in a form which is non-open and not mixed with the bolus.
